# EUROPEAN PATENT SPECIFICATION

(11) **EP 1 383 905 B1**
(45) Date of publication and mention of the grant of the patent: **16.07.2008**
(21) Application number: 02702397.7
(22) Date of filing: 06.03.2002
(51) Int. Cl.: C12P 13/08

(54) **PROCESS FOR THE PRODUCTION OF L-AMINO ACIDS USING STRAINS OF THE FAMILY ENTEROBACTERIACEAE THAT CONTAIN AN ATTENUATED ACEA GENE**
VERFAHREN ZUR HERSTELLUNG VON L-AMINOSÄUREN, UNTER VERWENDUNG VON STÄMMEN AUS DER FAMILIE DER ENTEROBACTERIACEAE, WELCHE EIN ATTENUIERTES ACEA-GEN ENTHALTEN
PROCEDE DE PRODUCTION DE L-AMINO ACIDES AU MOYEN DE SOUCHES DE LA FAMILLE ENTEROBACTERIACEAE CONTENANT UN GENE ACEA ATTENUE

(30) Priority: 03.04.2001 DE 10116518
(43) Date of publication of application: 28.01.2004
(73) Proprietor: Evonik Degussa GmbH, 40474 Düsseldorf (DE)
(72) Inventor: RIEPING, Mechthild, 33619 Bielefeld (DE); HERMANN, Thomas, 33739 Bielefeld (DE)
(86) International application number: PCT/EP2002/002421
(87) International publication number: WO 2002/081722

(56) References cited:
- EP-A- 0 237 819
- EP-A- 0 643 135
- EP-A- 0 952 221
- EP-A- 0 955 368
- WO-A-99/53035
- WO-A-02/081698
- WO-A-02/081721
- US-A- 4 278 765
- US-A- 4 368 266
- MATSUOKA M ET AL.: "Isolation, hyperexpression, and sequencing of the aceA gene encoding isocitrate lyase in Escherichia coli" JOURNAL OF BACTERIOLOGY, vol. 170, no. 10, 1988, pages 4528-4536, XP008016923 ISSN: 0021-9193 cited in the application
- MICHAL G: "Biochemical pathways: an atlas of biochemistry and molecular biology" 1999 , JOHN WILEY & SONS INC. AND SPEKTRUM AKADEMISCHER VERLAG , NEW YORK - HEIDELBERG XP002240819 ISBN: 0-471-33130-9 figures 3.8-2, 3.9-1 figures 4.2-1, 4.5-1 and 4.5-2
- KRAEMER R: "Genetic and physiological approaches for the production of amino acids" JOURNAL OF BIOTECHNOLOGY, vol. 45, no. 1, 1996, pages 1-21, XP002178648 ISSN: 0168-1656
- DATABASE WPI Section Ch, Week 199148 Derwent Publications Ltd., London, GB; Class B05, AN 1991-351136 XP002241222 & JP 03 236786 A (KYOWA HAKKO KOGYO KK), 22 October 1991 (1991-10-22)
- JETTEN M S M ET AL.: "Recent advances in the physiology and genetics of amino acid-producing bacteria." CRC CRITICAL REVIEWS IN BIOTECHNOLOGY, vol. 15, no. 1, 1995, pages 73-103, XP000613291 ISSN: 0738-8551
- CHUNG T ET AL.: "Glyoxylate bypass operon of Escherichia coli: cloning and determination of the functional map." JOURNAL OF BACTERIOLOGY, vol. 170, no. 1, January 1988 (1988-01), pages 386-392, XP008015355 ISSN: 0021-9193
- SAWERS G: "The anaerobic degradation of L-serine and L-threonine in enterobacteria: networks of pathways and regulatory signals" ARCHIVES OF MICROBIOLOGY, vol. 171, no. 1, 1998, pages 1-5, XP002953871 ISSN: 0302-8933

## Description

### Field of the Invention

The present invention relates to a process for the production of L-lysine and L-threonine, using strains of the family Enterobacteriaceae in which the aceA gene is switched off.

### Prior Art

L-amino acids, in particular L-threonine, are used in human medicine and in the pharmaceutical industry, in the foodstuffs industry, and most especially in animal nutrition.

It is known to produce L-amino acids by fermentation of strains of Enterobacteriaceae, in particular Escherichia coli (E. coli) and Serratia marcescens. On account of their great importance efforts are constantly being made to improve processes for producing the latter. Process improvements may relate to fermentation technology measures, such as for example stirring and provision of oxygen, or the composition of the nutrient media, such as for example the sugar concentration during the fermentation, or the working-up to the product form, for example by ion exchange chromatography, or the intrinsic performance properties of the microorganism itself.

Methods comprising mutagenesis, selection and mutant choice are employed in order to improve the performance properties of these microorganisms. In this way strains are obtained that are resistant to antimetabolites, such as for example the threonine analogue α-amino-β-hydroxyvaleric acid (AHV) or are auxotrophic for regulatorily important metabolites, and that produce L-amino acids such as for example L-threonine.

Methods of recombinant DNA technology have also been used for some years in order to improve strains of the family Enterobacteriaceae producing L-amino acids, by amplifying individual amino acid biosynthesis genes and investigating their effect on production.

### Object of the Invention

The object of the invention is to provide new measures for the improved production of L-lysine and L-threonine.

### Summary of the Invention

The invention provides a process for the production of L-lysine and L-threonine, using microorganisms of the family Enterobacteriaceae that in particular already produce L-amino acids and in which the nucleotide sequence coding for the aceA gene is switched off.

### Detailed Description of the Invention

Where L-amino acids or amino acids are mentioned hereinafter, this is understood to mean one or more amino acids including their salts, selected from L-threonine, L-methionine, L-isoleucine, L-leucine, L-tyrosine, and L-lysine. L-threonine is particularly preferred.

The term "attenuation" describes in this connection the reduction or switching off of the intracellular activity of one or more enzymes (proteins) in a microorganism that are coded by the corresponding DNA, by using for example a weak promoter or a gene or allele that codes for a corresponding enzyme with a low activity and/or that inactivates the corresponding enzyme (protein) or gene, and optionally combining these measures.

By means of these attenuation measures the activity or concentration of the corresponding protein is generally reduced to 0 to 75%, 0 to 50%, 0 to 25%, 0 to 10% or 0 to 5% of the activity or concentration of the wild type protein, or the activity or concentration of the protein in the initial microorganism.

The invention provides a process for the production of L-amino-acids, chosen from the group L-threonine and L-lysine, wherein the following steps are carried out:
a) fermentation of microorganisms of the family Enterobacteriaceae producing the desired L-amino acid,
b) enrichment of the L-amino acid in the medium or in the cells of the microorganisms, and
c) isolation of the L-amino acid, in which optionally constituents of the fermentation broth and/or the biomass in its entirety or portions thereof remain in the product,
wherein in the microorganism compared to the initial microorganism the expression of the aceA gene or the expression of nucleotide sequences coding for the gene product of said gene, the isocitrate lyase, is switched off.

The microorganisms that are the subject of the present invention can produce L-amino acids from glucose, sucrose, lactose, fructose, maltose, molasses, optionally starch, optionally cellulose or from glycerol and ethanol. The microorganisms are members of the family Enterobacteriaceae selected from the genera Escherichia, Erwinia, Providencia and Serratia. The genera Escherichia and Serratia are preferred. In the case of the genus Escherichia the species Escherichia coli may in particular be mentioned, and in the case of the genus Serratia the species Serratia marcescens may in particular be mentioned.

Suitable strains of the genus Escherichia, in particular those of the species Escherichia coli, that produce in particular L-threonine, include for example:
Escherichia coli TF427
Escherichia coli H4578
Escherichia coli KY10935
Escherichia coli VNIIgenetika MG442
Escherichia coli VNIIgenetika M1
Escherichia coli VNIIgenetika 472T23
Escherichia coli BKIIM B-3996
Escherichia coli kat 13
Escherichia coli KCCM-10132

Suitable strains of the genus Serratia, in particular of the species Serratia marcescens, that produce L-threonine include for example:
Serratia marcescens HNr21
Serratia marcescens TLr156
Serratia marcescens T2000

Strains of the family of Enterobacteriaceae producing L-threonine preferably have, *inter alia,* one or more of the genetic or phenotype features selected from the following group: resistance to α-amino-β-hydroxyvaleric acid, resistance to thialysine, resistance to ethionine, resistance to α-methylserine, resistance to diaminosuccinic acid, resistance to α-aminobutyric acid, resistance to borrelidin, resistance to rifampicin, resistance to valine analogues such as for example valine hydroxamate, resistance to purine analogues such as for example 6-dimethylaminopurine, need for L-methionine, optionally partial and compensable need for L-isoleucine, need for meso-diaminopimelic acid, auxotrophy with regard to threonine-containing dipeptides, resistance to L-threonine, resistance to L-homoserine, resistance to L-lysine, resistance to L-methionine, resistance to L-glutamic acid, resistance to L-aspartate, resistance to L-leucine, resistance to L-phenylalanine, resistance to L-serine, resistance to L-cysteine, resistance to L-valine, sensitivity to fluoropyruvate, defective threonine dehydrogenase, optionally ability to utilize sucrose, enhancement of the threonine operon, enhancement of homoserine dehydrogenase, I-aspartate kinase I, preferably of the feedback-resistant form, enhancement of homoserine kinase, enhancement of threonine synthase, enhancement of aspartate kinase, optionally of the feedback-resistant form, enhancement of aspartate semialdehyde dehydrogenase, enhancement of phosphoenol pyruvate carboxylase, optionally of the feedback-resistant form, enhancement of phosphoenol pyruvate synthase, enhancement of transhydrogenase, enhancement of the RhtB gene product, enhancement of the RhtC gene product, enhancement of the YfiK gene product, enhancement of a pyruvate carboxylase, and attenuation of acetic acid formation.

It has now been found that microorganisms of the family Enterobacteriaceae after switching off the aceA gene, produce L-lysine and L-threonine in an improved way.

The nucleotide sequences of the Escherichia coli genes belong to the prior art and may also be obtained,from the genome sequence of Escherichia coli published by Blattner et al. (Science 277, 1453 - 1462 (1997)).

The aceA gene is described *inter alia* by the following data:

| | |
|---|---|
| Designation: | Isocitrate lyase |
| EC-No.: | 4.1.3.1 |
| Reference: | Matsuoko and McFadden; Journal of Bacteriology 170, 4528-4536 (1988) |
| Accession No.: | AE000474 |

Apart from the described aceA gene, alleles of the gene may be used that result from the degeneracy of the genetic code or from functionally neutral sense mutations, the activity of the protein not being substantially altered.

The gene expression may be reduced by suitable culture conditions, by genetic alteration (mutation) of the signal structures of the gene expression, or also by antisense-RNA techniques. Signal structures of the gene expression are for example repressor genes, activator genes, operators, promoters, attenuators, ribosome-binding sites, the start codon and terminators. The person skilled in the art may find relevant information in, *inter alia,* articles by Jensen and Hammer (Biotechnology and Bioengineering 58: 191-195 (1998)), by Carrier and Keasling (Biotechnology Progress 15, 58-64 (1999), Franch and Gerdes (Current Opinion in Microbiology 3, 159-164 (2000)) and in known textbooks of genetics and molecular biology, such as for example the textbook by Knippers ("Molekulare Genetik", 6th Edition, Georg Thieme Verlag, Stuttgart, Germany, 1995) or that by Winnacker ("Gene and Klone", VCH Verlagsgesellschaft, Weinheim, Germany, 1990).

Mutations that lead to a change or reduction of the catalytic properties of enzyme proteins are known from the prior art. As examples there may be mentioned the work by Qiu and Goodman (Journal of Biological Chemistry 272: 8611-8617 (1997)), Yano et al. (Proceedings of the National Academy of Sciences, USA 95, 5511-5515 (1998), Wente and Schachmann (Journal of Biological Chemistry 266, 20833-20839 (1991). Detailed information may be obtained from known textbooks on genetics and molecular biology, such as for example that by Hagemann ("Allgemeine Genetik", Gustav Fischer Verlag, Stuttgart, 1986).

Suitable mutations include transitions, transversions, insertions and deletions. Depending on the action of the amino acid exchange on the enzyme activity, one speaks of missense mutations or nonsense mutations. Insertions or deletions of at least one base pair in a gene lead to frame shift mutations, which in turn lead to the incorporation of false amino acids or the premature termination of a translation. If as a result of the mutation a stop codon is formed in the coding region, this also leads to a premature termination of the translation. Deletions of several codons typically lead to a complete disruption of the enzyme activity. Details regarding the production of such mutations belong to the prior art and may be obtained from known textbooks on genetics and molecular biology, such as for example the textbook by Knippers ("Molekulare Genetik", 6th Edition, Georg Thieme Verlag, Stuttgart, Germany, 1995), that by Winnacker ("Gene und Klone", VCH Verlagsgesellschaft, Weinheim, Germany, 1990) or that by Hagemann ("Allgemeine Genetik", Gustav Fischer Verlag, Stuttgart, 1986).

Suitable mutations in the genes such as for example deletion mutations may be incorporated by gene and/or allele exchange in suitable strains.

A conventional method is the method of gene exchange by means of a conditionally replicating pSC101 derivate pMAK705 described by Hamilton et al. (Journal of Bacteriology 171, 4617 - 4622 (1989)). Other methods described in the prior art, such as for example that of Martinez-Morales et al. (Journal of Bacteriology 181, 7143-7148 (1999)) or that of Boyd et al. (Journal of Bacteriology 182, 842-847 (2000)) may likewise be used.

It is also possible to transfer mutations in the respective genes or mutations relating to the expression of the relevant genes, by conjugation or transduction into various strains.

Furthermore for the production of L-lysine and L-threonine, using strains of the family Enterobacteriaceae it may be advantageous in addition to the switching off of the aceA gene also to overexpress one or more enzymes of the known threonine biosynthesis pathway or enzymes of anaplerotic metabolism or enzymes for the production of reduced nicotinamide-adenine-dinucleotide phosphate.

The term "enhancement" describes in this connection the raising of the intracellular activity of one or more enzymes or proteins in a microorganism that are coded by the corresponding DNA, by for example increasing the number of copies of the gene or genes, using a strong promoter or a gene that codes for a corresponding enzyme or protein having a high activity, and optionally by combining these measures.

By means of the overexpression, the activity or concentration of the corresponding protein is in general raised by at least 10%, 25%, 50%, 75%, 100%, 150%, 200%, 300%, 400% or 500%, at most up to 1000% or 2000% referred to that of the wild type protein and/or the activity or concentration of the protein in the initial microorganism.

Thus, one or more of the genes selected from the following group may for example by simultaneously overexpressed:
● the thrABC operon coding for aspartate kinase, homoserine dehydrogenase, homoserine kinase and threonine synthase (US-A-4,278,765),
● the pyc gene coding for pyruvate carboxylase (DE-A-19 831 609),
● the pps gene coding for phosphoenol pyruvate synthase (Molecular and General Genetics 231:332 (1992)),
● the ppc gene coding for phosphoenol pyruvate carboxylase (Gene 31:279-283 (1984)),
● the genes pntA and pntB coding for transhydrogenase (European Journal of Biochemistry 158:647-653 (1986)),
● the gene rhtB imparting homoserine resistance (EP-A-0 994 190),
● the mqo gene coding for malate:quinone oxidoreductase (DE 100 348 33.5),
● the gene rhtC imparting threonine resistance (EP-A-1 013 765), and
● the thrE gene of Corynebacterium glutamicum coding for threonine export (DE 100 264 94.8).

The use of endogenous genes is in general preferred. The term "endogenous genes" or "endogenous nucleotide sequences" is understood to mean the genes or nucleotide sequences present in the population of a species.

Furthermore for the production of L-lysine and L-threonine, it may be advantageous in addition to the switching off of the aceA gene also to switch off one or more of the genes selected from the following group:
● the tdh gene coding for threonine dehydrogenase (Ravnikar and Somerville, Journal of Bacteriology 169, 4716-4721 (1987)),
● the mdh gene coding for malate dehydrogenase (E.C. 1.1.1.37) (Vogel et al., Archives in Microbiology 149, 36-42 (1987)),
● the gene product of the open reading frame (orf) yjfA (Accession Number AAC77180 of the National Center for Biotechnology Information (NCBI, Bethesda, MD, USA),
● the gene product of the open reading frame (orf) ytfP (Accession Number AAC77179 des National Center for Biotechnology Information (NCBI, Bethesda, MD, USA),
● the pckA gene coding for the enzyme phosphoenol pyruvate carboxykinase (Medina et al. (Journal of Bacteriology 172, 7151-7156 (1990)),
● the poxB gene coding for pyruvate oxidase (Grabau and Cronan (Nucleic Acids Research 14 (13), 5449-5460 (1986)),
● the dgsA gene coding for the regulator of the phosphotransferase system (Hosono et al., Bioscience, Biotechnology and Biochemistry 59, 256-251 (1995) and Accession No.: AE000255), and
● the fruR gene coding for the fructose repressor (Jahreis et al., Molecular and General Genetics 226, 332-336 (1991) and Accession No.: AE000118)

Furthermore for the production of L-lysine and L-threonine, it may be advantageous in addition to the switching off of the aceA gene also to switch off undesirable secondary reactions (Nakayama: "Breeding of Amino Acid Producing Microorganisms", in: Overproduction of Microbial Products, Krumphanzl, Sikyta, Vanek (eds.), Academic Press, London, UK, 1982).

The microorganisms produced according to the invention may be cultivated in a batch process (batch cultivation), in a fed batch process (feed process) or in a repeated fed batch process (repetitive feed process). A summary of known cultivation methods is described in the textbook by Chmiel (Bioprozesstechnik 1. Einführung in die Bioverfahrenstechnik (Gustav Fischer Verlag, Stuttgart, 1991)) or in the textbook by Storhas (Bioreaktoren and periphere Einrichtungen (Vieweg Verlag, Brunswick /Wiesbaden, 1994)).

The culture medium to be used must appropriately satisfy the requirements of the respective strains. Descriptions of culture media of various microorganisms are contained in the handbook "Manual of Methods for General Bacteriology" of the American Society for Bacteriology (Washington D.C., USA, 1981).

As carbon sources, sugars and carbohydrates such as for example glucose, sucrose, lactose, fructose, maltose, molasses, starch and optionally cellulose, oils and fats such as for example soya bean oil, sunflower oil, groundnut oil and coconut oil, fatty acids such as for example palmitic acid, stearic acid and linoleic acid, alcohols such as for example glycerol and ethanol, and organic acids such as for example acetic acid, may be used. These substances may be used individually or as a mixture.

As nitrogen source, organic nitrogen-containing compounds such as peptones, yeast extract, meat extract, malt extract, maize starch water, soya bean flour and urea or inorganic compounds such as ammonium sulfate, ammonium chloride, ammonium phosphate, ammonium carbonate and ammonium nitrate may be used. The nitrogen sources may be used individually br as a mixture.

As phosphorus source, phosphoric acid, potassium dihydrogen phosphate or dipotassium hydrogen phosphate or the corresponding sodium-containing salts may be used. The culture medium must furthermore contain salts of metals, such as for example magnesium sulfate or iron sulfate, that are necessary for growth. Finally, essential growth promoters such as amino acids and vitamins may be used in addition to the aforementioned substances. Apart from these, suitable precursors may be added to the culture medium. The aforementioned starting substances may be added to the culture in the form of a single batch or may be metered in in an appropriate manner during the cultivation.

In order to regulate the pH of the culture basic compounds such as sodium hydroxide, potassium hydroxide, ammonia or ammonia water, or acidic compounds such as phosphoric acid or sulfuric acid are used as appropriate. In order to control foam formation antifoaming agents such as for example fatty acid polyglycol esters may be used. In order to maintain the stability of plasmids, suitable selectively acting substances, for example antibiotics, may be added to the medium. In order to maintain aerobic conditions, oxygen or oxygen-containing gas mixtures such as for example air are fed into the culture. The temperature of the culture is normally 25°C to 45°C, and preferably 30°C to 40°C. Cultivation is continued until a maximum amount of L-amino acids (or L-threonine) has been formed. This target is normally achieved within 10 hours to 160 hours.

The L-amino acids may be analyzed by anion exchange chromotography followed by ninhydrin derivation, as described by Spackman et al. (Analytical Chemistry, 30, (1958), 1190), or by reversed phase HPLC, as described by Lindroth et al. (Analytical Chemistry (1979) 51: 1167-1174).

The process according to the invention can be used for the enzymatic production of L-amino acids, such as for example L-threonine, and L-lysine, in particular L-threonine.

A pure culture of the Escherichia coli K-12 strain DH5α/pMAK705 was filed as DSM 13720 on 08 September 2000 at the German Collection for Microorganisms and Cell Cultures (DSMZ, Brunswick, Germany) according to the Budapest Convention.

The present invention is described in more detail hereinafter with the aid of examples of implementation.

The isolation of plasmid DNA from Escherichia coli as well as all techniques for the restriction, Klenow treatment and alkaline phosphatase treatment are carried out according to Sambrook et al. (Molecular Cloning - A Laboratory Manual (1989) Cold Spring Harbor Laboratory Press). The transformation of Escherichia coli is, unless otherwise described, carried out according to Chung et al. (Proceedings of the National Academy of Sciences of the United States of America, USA (1989) 86: 2172-2175).

The incubation temperature in the production of strains and transformants is 37°C. In the gene exchange process according to Hamilton et al., temperatures of 30°C and 44°C are used.

### Example 1

### Construction of the deletion mutation of the aceA gene.

Parts of the gene regions lying upstream and downstream of the aceA gene are amplified from Escherichia coli K12 using the polymerase chain reaction (PCR) as well as synthetic oligonucleotides. Starting from the nucleotide sequence of the aceBAK operon in E. coli K12 MG1655 DNA (SEQ ID No. 1) the following PCR primers are synthesized (MWG Biotech, Ebersberg, Germany):
aceA'5'-1: 5' - ATGCTTACTCACGCCTGTTG - 3' (SEQ ID No. 3)
aceA'5'-2: 5' - CATGTGCAGATGCTCCATAG - 3' (SEQ ID No. 4)
aceA'3'-1: 5' - CAACAACAACCGTTGCTGAC - 3' (SEQ ID No. 5)
aceA'3'-2: 5' - CAGTTCGTTCGCCACCTGTA - 3' (SEQ ID No. 6)

The chromosomal E. coli K12 MG1655 DNA used for the PCR is isolated according to the manufacturer's instructions using "Qiagen Genomic-tips 100/G" (QIAGEN, Hilden, Germany). A ca. 700 bp large DNA fragment from the region lying upstream of the aceA gene (designated 'aceB) and a ca. 800 bp large DNA fragment from the region lying downstream of the aceA gene (designated aceK') can be amplified with the specific primers under standard PCR conditions (Innis et al. (1990) PCR Protocols. A guide to methods and applications, Academic Press) with Taq DNA polymerase (Gibco-BRL, Eggenstein, Germany). The PCR products are ligated according to the manufacturer's instructions in each case with the vector pCR2.1TOPO (TOPO TA Cloning Kit, Invitrogen, Groningen, Netherlands) and transformed in the E. coli strain TOP10F'. The selection of plasmid-carrying cells is carried out on LB agar to which 50 µg/ml of ampicillin has been added. After the plasmid DNA isolation the vector pCR2.1TOPO'aceB is cleaved with the restriction enzymes EcoRV and SpeI, and the 'aceB fragment after separation in 0.8% agarose gel is isolated using the QIAquick Gel Extraction Kit (QIAGEN, Hilden, Germany). After the plasmid DNA isolation the vector pCR2.1TOPOaceK' is cleaved with the enzymes Ecl136II. and SpeI and ligated with the isolated 'aceB fragment. The E. coli strain DH5α is transformed with the ligation batch and plasmid-carrying cells are selected on LB agar to which 50 µg/ml of ampicillin has been added. After the plasmid DNA isolation, those plasmids in which the mutagenic DNA sequence shown in SEQ ID No. 7 is present in cloned form are detected by control cleavage with the enzymes HindIII and XbaI. One of the plasmids is designated pCR2.1TOPOΔaceA.

### Example 2

### Construction of the exchange vector pMAK705ΔaceA

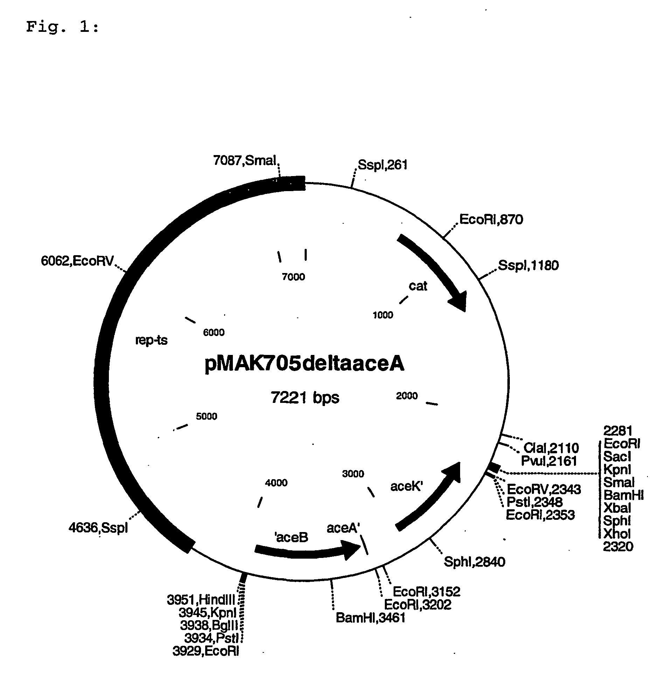

The aceBAK allele described in Example 1 is isolated from the vector pCR2.1TOPOΔaceA after restriction with the enzymes HindIII and XbaI and separation in 0.8% agarose gel, and is ligated with the plasmid pMAK705 (Hamilton et al. (1989) Journal of Bacteriology 171, 4617 - 4622), that had been digested with the enzymes HindIII and XbaI. The ligation batch is transformed in DH5α and plasmid-carrying cells are selected on LB agar to which 20 µg/ml of chloramphenicol have been added. The successful cloning is detected after plasmid DNA isolation and cleavage with the enzymes BamHI, KpnI, SphI, SpeI and PstI. The resultant exchange vector pMAK705ΔaceA (= pMAK705deltaaceA) is shown in Fig. 1.

### Example 3

### Site-specific mutagenesis of the aceA gene in the E. coli strain MG442

The E. coli strain MG442 producing L-threonine is described in patent specification US-A- 4,278,765 and is filed as CMIM B-1628 at the Russian National Collection for Industrial Microorganisms (VKPM, Moscow, Russia).

For the exchange of the chromosomal aceA gene by the plasmid-coded deletion construct, MG442 is transformed with the plasmid pMAK705ΔaceA. The gene exchange is carried out by the selection process described by Hamilton et al. (1989) Journal of Bacteriology 171, 4617 - 4622) and is verified by standard PCR methods (Innis et al. (1990) PCR Protocols. A guide to methods and applications, Academic Press) with the following oligonucleotide primers:
aceA'5'-1: 5' - ATGCTTACTCACGCCTGTTG - 3' (SEQ ID No. 3)
aceA'3'-2: 5' - CAGTTCGTTCGCCACCTGTA - 3' (SEQ ID No. 6)

The resultant strain is designated MG442ΔaceA.

### Example 4

### Production of L-threonine using the strain MG442ΔaceA

MG442ΔaceA is cultivated on minimal medium having the following composition: 3.5 g/l Na₂HPO₄·2H₂O, 1.5 g/l KH₂PO₄, 1 g/l NH₄Cl, 0.1 g/l MgSO₄·7H₂O, 2 g/l glucose and 20 g/l agar. The formation of L-threonine is checked in batch cultures of 10 ml that are contained in 100 ml Erlenmeyer flasks. For this, 10 ml of preculture medium of the following composition: 2 g/l yeast extract, 10 g/l (NH₄)₂SO₄, 1 g/l KH₂PO₄, 0.5 g/l MgSO₄·7H₂O, 15 g/l CaCO₃, 20 g/l glucose are inoculated and incubated for 16 hours at 37°C and 180 rpm in an ESR incubator from Kühner AG (Birsfelden, Switzerland). 250 µl of this preculture are reinoculated in 10 ml of production medium (25 g/l (NH₄)₂SO₄, 2 g/l KH₂PO₄, 1 g/l MgSO₄·7H₂O, 0.03 g/l FeSO₄·7H₂O, 0.018 g/l MnSO₄·1H₂O, 30 g/l CaCO₃ and 20 g/l glucose) and incubated for 48 hours at 37°C. After incubation the optical density (OD) of the culture suspension is measured with an LP2W photometer from the Dr. Lange company (Dusseldorf, Germany) at a measurement wavelength of 660 nm.

The concentration of formed L-threonine is then determined in the sterile-filtered culture supernatant using an amino acid analyzer from Eppendorf-BioTronik (Hamburg, Germany) by ion exchange chromatography and post-column reaction with ninhydrin detection.

The result of the test is given in Table 1.

**Table 1**

| Strain | OD (660 nm) | L-threonine g/l |
|---|---|---|
| MG442 | 6.0 | 1.5 |
| MG442ΔaceA | 6.2 | 1.9 |

### Brief Description of the Figure:

● Fig. 1: pMAK705ΔaceA ( = pMAK705deltaaceA)

Length data are given as approximate values. The abbreviations and acronyms used have the following meanings:
● cat: chloramphenicol resistance gene
● rep-ts: temperature-sensitive replication region of the plasmid pSC101
● 'aceB: part of the 3' region of the aceB gene
● aceA': ATG start codon of the aceA gene
● aceK': part of the 5' region of the aceK gene

The abbreviations for the restriction enzymes have the following meanings:
● BamHI: restriction endonuclease from Bacillus amyloliquefaciens
● BglII: restriction endonuclease from Bacillus globigii
● ClaI: restriction endonuclease from Caryphanon latum
● EcoRI: restriction endonuclease from Escherichia coli
● EcoRV: restriction endonuclease from Escherichia coli
● HindIII: restriction endonuclease from Haemophilus influenzae
● KpnI: restriction endonuclease from Klebsiella pneumoniae
● PstI: restriction endonuclease from Providencia stuartii
● PvuI: restriction endonuclease from Proteus vulgaris
● SacI: restriction endonuclease from Streptomyces achromogenes
● SalI: restriction endonuclease from Streptomyces albus
● SmaI: restriction endonuclease from Serratia marcescens
● SphI: restriction endonuclease from Streptomyces phaeochromogenes
● SspI: restriction endonuclease from Sphaerotilus species
● XbaI: restriction endonuclease from Xanthomonas badrii
● XhoI: restriction endonuclease from Xanthomonas holcicola

## Claims

1. Process for the production of L-amino-acids, chosen from the group L-threonine and L-lysine, wherein the following steps are carried out:
a) fermentation of microorganisms of the family Enterobacteriaceae producing the desired L-amino acid,
b) enrichment of the L-amino acid in the medium or in the cells of the microorganisms, and
c) isolation of the L-amino acid, in which optionally constituents of the fermentation broth and/or the biomass in its entirety or portions thereof remain in the product,
wherein in the microorganism compared to the initial microorganism the expression of the aceA gene or the expression of nucleotide sequences coding for the gene product of said gene, the isocitrate lyase, is switched off.

2. Process according to claim 1, wherein, for the production of L-threonine, microorganisms of the family Enterobacteriaceae are fermented in which at the same time one or more of the genes selected from the following group is or are over-expressed:
2.1 the thrABC operon coding for aspartate kinase, homoserine dehydrogenase, homoserine kinase and threonine synthase,
2.2 the pyc gene coding for pyruvate carboxylase,
2.3 the pps gene coding for phosphoenol pyruvate synthase,
2.4 the ppc gene coding for phosphoenol pyruvate carboxylase,
2.5 the genes pntA and pntB coding for transhydrogenase,
2.6 the Escherichia coli rhtB gene coding for a protein imparting homoserine resistance,
2.7 the mqo gene coding for malate:quinone oxidoreductase,
2.8 the Escherichia coli rhtC gene coding for a protein imparting threonine resistance, and
2.9 the thrE gene coding for threonine export.

3. Process according to claim 1, wherein, for the production of L-threonine, microorganisms of the family Enterobacteriaceae are fermented in which at the same time the expression of one or more of the genes selected from the following group is or are switched off:
3.1 the tdh gene coding for threonine dehydrogenase,
3.2 the mdh gene coding for malate dehydrogenase,
3.3 the gene product of the open reading frame (orf) yjfA of E. coli, when said microorganism is Escherichia coli,
3.4 the gene product of the open reading frame (orf) ytfP of E. coli, when said microorganism is Escherichia coli,
3.5 the pckA gene coding for phosphoenol pyruvate carboxykinase, and
3.6 the poxB gene coding for pyruvate oxidase.

4. Microorganisms of the Enterobacteriaceae family, producing L-threonine, wherein at least:
a) the thrABC operon which genes code for aspartate kinase, homoserine dehydrogenase, homoserine kinase and threonine synthase is over-expressed, and
b) the expression of the aceA gene or nucleotide sequences coding for the gene product of said gene is switched off.

5. Microorganisms of the Enterobacteriaceae family, producing L-lysine, wherein at least:
a) the expression of the pckA gene coding for phosphoenol pyruvate carboxykinase, and
b) the expression of the aceA gene or nucleotide sequences coding for the gene product of said gene
is switched off.

6. Microorganisms according to claim 4 or 5, wherein the microorganisms originate from the genus Escherichia.

7. Microorganisms according to claim 6, wherein the microorganisms originate from the species E. coli.

## Patentansprüche

1. Verfahren zur Herstellung von L-Aminosäuren, ausgewählt aus der Gruppe L-Threonin und L-Lysin, bei dem man die folgenden Schritte durchführt:
a) Fermentation von Mikroorganismen der Familie Enterobacteriaceae, die die gewünschte L-Aminosäure produzieren,
b) Anreicherung der L-Aminosäure im Medium oder in den Zellen der Mikroorganismen und
c) Isolierung der L-Aminosäure, wobei gegebenenfalls Bestandteile der Fermentationsbrühe und/oder die Biomasse insgesamt oder teilweise im Produkt verbleiben,
wobei die Expression des aceA-Gens oder die Expression der für das Genprodukt des Gens, die Isocitrat-Lyase, codierenden Nukleotidsequenzen in dem Mikroorganismus im Vergleich zu dem ursprünglichen Mikroorganismus ausgeschaltet ist.

2. Verfahren gemäß Anspruch 1, wobei man zur Herstellung von L-Threonin Mikroorganismen der Familie Enterobacteriaceae fermentiert, in denen gleichzeitig eines oder mehrere der Gene, ausgewählt aus der folgenden Gruppe, überexprimiert wird bzw. werden:
2.1 das für Aspartatkinase, Homoserin-Dehydrogenase, Homoserinkinase und Threonin-Synthase codierende thrABC-Operon,
2.2 das für die Pyruvat-Carboxylase codierende pyc-Gen,
2.3 das für die Phosphoenolpyruvat-Synthase codierende pps-Gen,
2.4 das für die Phosphoenolpyruvat-Carboxylase codierende ppc-Gen,
2.5 die für Transhydrogenase codierenden Gene pntA und pntB,
2.6 das für ein Homoserin-Resistenz verleihendes Protein codierende rhtB-Gen aus Escherichia coli,
2.7 das für die Malat:Chinon-Oxidoreduktase codierende mqo-Gen,
2.8 das für ein Threonin-Resistenz verleihendes Protein codierende rhtC-Gen aus Escherichia coli und
2.9 das für Threonin-Export codierende thrE-Gen.

3. Verfahren gemäß Anspruch 1, wobei man zur Herstellung von L-Threonin Mikroorganismen der Familie Enterobacteriaceae fermentiert, in denen gleichzeitig die Expression eines oder mehrerer Gene, ausgewählt aus der nachfolgenden Gruppe, ausgeschaltet ist:
3.1 das für die Threonin-Dehydrogenase codierende tdh-Gen,
3.2 das für die Malat-Dehydrogenase codierende mdh-Gen,
3.3 das Genprodukt des offenen Leserasters (orf) yjfA aus E. coli, wenn es sich bei dem Mikroorganismus um Escherichia coli handelt,
3.4 das Genprodukt des offenen Leserasters (orf) ytfP aus E. coli, wenn es sich bei dem Mikroorganismus um Escherichia coli handelt,
3.5 das für die Phosphoenolpyruvat-Carboxykinase codierende pckA-Gen und
3.6 das für die Pyruvat-Oxidase codierende poxB-Gen.

4. Mikroorganismen der Familie Enterobacteriaceae, die L-Threonin produzieren, wobei wenigstens:
a) das thrABC-Operon, dessen Gene für Aspartatkinase, Homoserin-Dehydrogenase, Homoserinkinase und Threonin-Synthase codieren, überexprimiert wird und
b) die Expression des aceA-Gens oder von für das Genprodukt des Gens codierenden Nukleotidsequenzen ausgeschaltet ist.

5. Mikroorganismen der Familie Enterobacteriaceae, die L-Lysin produzieren, wobei wenigstens:
a) die Expression des für die Phosphoenolpyruvat-Carboxykinase codierenden pckA-Gens sowie
b) die Expression des aceA-Gens oder von für das Genprodukt des Gens codierenden Nukleotidsequenzen ausgeschaltet ist.

6. Mikroorganismen gemäß Anspruch 4 oder 5, wobei die Mikroorganismen aus der Gattung Escherichia stammen.

7. Mikroorganismen gemäß Anspruch 6, wobei die Mikroorganismen aus der Spezies E. coli stammen.

## Revendications

1. Procédé de production d'acides L-aminés, choisis dans le groupe comprenant la L-thréonine et la L-lysine, **caractérisé en ce que** les étapes suivantes sont effectuées :
a) la fermentation de microorganismes de la famille des Enterobacteriaceae produisant l'acide L-aminé désiré,
b) l'enrichissement de l'acide L-aminé dans le milieu ou dans les cellules des microorganismes, et
c) l'isolement de l'acide L-aminé, où éventuellement les constituants du bouillon de fermentation et/ou la biomasse dans son intégralité ou des portions de celle-ci restent dans le produit,
**en ce que**, dans le microorganisme par rapport au microorganisme initial, l'expression du gène aceA ou l'expression des séquences nucléotidiques codant pour le produit génique dudit gène, l'isocitrate lyase, est éteinte.

2. Procédé selon la revendication 1, **caractérisé en ce que**, pour la production de la L-thréonine, des microorganismes de la famille des Enterobacteriaceae sont mis en fermentation, dans lesquels, en même temps, un ou plusieurs des gènes choisis dans le groupe suivant sont surexprimés :
2.1 l'opéron thrABC codant pour l'aspartate kinase, l'homosérine déshydrogénase, l'homosérine kinase et la thréonine synthase,
2.2 le gène pyc codant pour la pyruvate carboxylase,
2.3 le gène pps codant pour la phosphoénol pyruvate synthase,
2.4 le gène ppc codant pour la phosphoénol pyruvate carboxylase,
2.5 les gènes pntA et pntB codant pour la transhydrogénase,
2.6 le gène rhtB d'Escherichia coli codant pour une protéine donnant la résistance à l'homosérine,
2.7 le gène mqo codant pour la malate:quinone oxydoréductase,
2.8 le gène rhtC d'Escherichia coli codant pour une protéine donnant la résistance à la thréonine, et
2.9 le gène thrE codant pour l'exportation de la thréonine.

3. Procédé selon la revendication 1, **caractérisé en ce que**, pour la production de la L-thréonine, des microorganismes de la famille des Enterobacteriaceae sont mis en fermentation, dans lesquels, en même temps, l'expression d'un ou plusieurs des gènes choisis dans le groupe suivant est éteinte :
3.1 le gène tdh codant pour la thréonine déshydrogénase,
3.2 le gène mdh codant pour la malate déshydrogénase,
3.3 le produit du gène du cadre de lecture ouvert (orf) yjfA d'E. coli, lorsque ledit microorganisme est l'Escherichia coli,
3.4 le produit du gène du cadre de lecture ouvert (orf) ytfP d'E. coli, lorsque ledit microorganisme est l'Escherichia coli,
3.5 le gène pckA codant pour la phosphoénol pyruvate carboxykinase, et
3.6 le gène poxB codant pour la pyruvate oxydase.

4. Microorganismes de la famille des Enterobacteriaceae, produisant la L-thréonine, **caractérisés en ce qu'**au moins :
a) l'opéron thrABC, dont les gènes codent pour l'aspartate kinase, l'homosérine déshydrogénase, l'homosérine kinase et la thréonine synthase, est surexprimé, et
b) l'expression du gène aceA ou des séquences nucléotidiques codant pour le produit génique dudit gène est éteinte.

5. Microorganismes de la famille des Enterobacteriaceae, produisant la L-lysine, **caractérisés en ce qu'**au moins :
a) l'expression du gène pckA codant pour la phosphoénol pyruvate carboxykinase, et
b) l'expression du gène aceA ou des séquences nucléotidiques codant pour le produit génique dudit gène,
sont éteintes.

6. Microorganismes selon la revendication 4 ou 5, **caractérisés en ce que** les microorganismes proviennent du genre Escherichia.

7. Microorganismes selon la revendication 6, **caractérisés en ce que** les microorganismes proviennent de l'espèce E. coli.


### SEQUENCE LISTING

<110> Degussa AG
<120> Process for the production of L-amino acids using strains of the family Enterobacteriaceae containing an attenuated aceA gene
<130> 010131 BT
<160> 7
<170> PatentIn version 3.1
<210> 1
   <211> 4855
   <212> DNA
   <213> Escherichia coli
<220>
   <221> CDS
   <222> (1632)..(2936)
   <223> aceA gene
<400> 1
<210> 2
   <211> 434
   <212> PRT
   <213> Escherichia coli
<400> 2
   Met Lys Thr Arg Thr Gln Gln Ile Glu Glu Leu Gln Lys Glu Trp Thr
   1               5                   10                  15
   Gln Pro Arg Trp Glu Gly Ile Thr Arg Pro Tyr Ser Ala Glu Asp Val
               20                  25                  30
   Val Lys Leu Arg Gly Ser Val Asn Pro Glu Cys Thr Leu Ala Gln Leu
           35                  40                  45
   Gly Ala Ala Lys Met Trp Arg Leu Leu His Gly Glu Ser Lys Lys Gly
       50                  55                  60
   Tyr Ile Asn Ser Leu Gly Ala Leu Thr Gly Gly Gln Ala Leu Gln Gln
   65                  70                  75                  80
   Ala Lys Ala Gly Ile Glu Ala Val Tyr Leu Ser Gly Trp Gln Val Ala
                   85                  90                  95
   Ala Asp Ala Asn Leu Ala Ala Ser Met Tyr Pro Asp Gln Ser Leu Tyr
               100                 105                 110
   Pro Ala Asn Ser Val Pro Ala Val Val Glu Arg Ile Asn Asn Thr Phe
           115                 120                 125
   Arg Arg Ala Asp Gln Ile Gln Trp Ser Ala Gly Ile Glu Pro Gly Asp
       130                 135                 140
   Pro Arg Tyr Val Asp Tyr Phe Leu Pro Ile Val Ala Asp Ala Glu Ala
   145                 150                 155                 160
   Gly Phe Gly Gly Val Leu Asn Ala Phe Glu Leu Met Lys Ala Met Ile
                   165                 170                 175
   Glu Ala Gly Ala Ala Ala Val His Phe Glu Asp Gln Leu Ala Ser Val
               180                 185                 190
   Lys Lys Cys Gly His Met Gly Gly Lys Val Leu Val Pro Thr Gln Glu
           195                 200                 205
   Ala Ile Gln Lys Leu Val Ala Ala Arg Leu Ala Ala Asp Val Thr Gly
       210                 215                 220
   Val Pro Thr Leu Leu Val Ala Arg Thr Asp Ala Asp Ala Ala Asp Leu
   225                 230                 235                 240
   Ile Thr Ser Asp Cys Asp Pro Tyr Asp Ser Glu Phe Ile Thr Gly Glu
                   245                 250                 255
   Arg Thr Ser Glu Gly Phe Phe Arg Thr His Ala Gly Ile Glu Gln Ala
               260                 265                 270
   Ile Ser Arg Gly Leu Ala Tyr Ala Pro Tyr Ala Asp Leu Val Trp Cys
           275                 280                 285
   Glu Thr Ser Thr Pro Asp Leu Glu Leu Ala Arg Arg Phe Ala Gln Ala
       290                 295                 300
   Ile His Ala Lys Tyr Pro Gly Lys Leu Leu Ala Tyr Asn Cys Ser Pro
   305                 310                 315                 320
   Ser Phe Asn Trp Gln Lys Asn Leu Asp Asp Lys Thr Ile Ala Ser Phe
                   325                 330                 335
   Gln Gln Gln Leu Ser Asp Met Gly Tyr Lys Phe Gln Phe Ile Thr Leu
   340 345 350
   Ala Gly Ile His Ser Met Trp Phe Asn Met Phe Asp Leu Ala Asn Ala
   355 360 365
   Tyr Ala Gln Gly Glu Gly Met Lys His Tyr Val Glu Lys Val Gln Gln
   370 375 380
   Pro Glu Phe Ala Ala Ala Lys Asp Gly Tyr Thr Phe Val Ser His Gln
   385 390 395 400
   Gln Glu Val Gly Thr Gly Tyr Phe Asp Lys Val Thr Thr Ile Ile Gln
   405 410 415
   Gly Gly Thr Ser Ser Val Thr Ala Leu Thr Gly Ser Thr Glu Glu Ser
   420 425 430
   Gln Phe
<210> 3
   <211> 20
   <212> DNA
   <213> Artificial sequence
<220>
   <221> Primer
   <222> (1)..(20)
   <223> aceA '5'-1
<400> 3
   atgcttactc acgcctgttg 20
<210> 4
   <211> 20
   <212> DNA
   <213> Artificial sequence
<220>
   <221> Primer
   <222> (1)..(20)
   <223> aceA '5'-2
<400> 4
   catgtgcaga tgctccatag 20
<210> 5
   <211> 20
   <212> DNA
   <213> Artificial sequence
<220>
   <221> Primer
   <222> (1)..(20)
   <223> aceA '3'-1
<400> 5
   caacaacaac cgttgctgac 20
<210> 6
   <211> 20
   <212> DNA
   <213> Artificial sequence
<220>
   <221> Primer
   <222> (1)..(20)
   <223> aceA '3'-2
<400> 6
   cagttcgttc gccacctgta 20
<210> 7
   <211> 1643
   <212> DNA
   <213> Escherichia coli
<220>
   <221> misc_feature
   <222> (1)..(33)
   <223> Technical DNA/ remainder polylinker sequence
<220>
   <221> misc_feature
   <222> (34)..(742)
   <223> Part of the 3' region of the aceB gene, intergenic region, ATG start codon of the aceA gene
<220>
   <221> misc_feature
   <222> (743)..(810)
   <223> Technical DNA/ remainder polylinker sequence
<220>
   <221> misc_feature
   <222> (811)..(1591)
   <223> Part of the 5' region of the aceK gene
<220>
   <221> misc_feature
   <222> (1592)..(1643)
   <223> Technical DNA/ remainder polylinker sequence
<400> 7
   agcttggtac cgagatctgc agaattcgcc cttatgctta ctcacgcctg ttgattaaaa 60
   cctgccataa acgcggtgct tttgcgatgg gcggcatggc ggcgtttatt ccgagcaaag 120
   atgaagagca caataaccag gtgctcaaca aagtaaaagc ggataaatcg ctggaagcca 180
   ataacggtca cgatggcaca tggatcgctc acccaggcct tgcggacacg gcaatggcgg 240
   tattcaacga cattctcggc tcccgtaaaa atcagcttga agtgatgcgc gaacaagacg 300
   cgccgattac tgccgatcag ctgctggcac cttgtgatgg tgaacgcacc gaagaaggta 360
   tgcgcgccaa cattcgcgtg gctgtgcagt acatcgaagc gtggatctct ggcaacggct 420
   gtgtgccgat ttatggcctg atggaagatg cggcgacggc tgaaatttcc cgtacctcga 480
   tctggcagtg gatccatcat caaaaaacgt tgagcaatgg caaaccggtg accaaagcct 540
   tgttccgcca gatgctgggc gaagagatga aagtcattgc cagcgaactg ggcgaagaac 600
   gtttctccca ggggcgtttt gacgatgccg cacgcttgat ggaacagatc accacttccg 660
   atgagttaat tgatttcctg accctgccag gctaccgcct gttagcgtaa accaccacat 720
   aactatggag catctgcaca tgaagggcga attccagcac actggcggcc gttactagta 780
   acggccgcca gtgtgctgga attcgccctt caacaacaac cgttgctgac tgtaggccgg 840
   ataaggcgtt cacgccgcat ccggcaatcg gtgcacgatg cctgatgcga cgcttgcgcg 900
   tcttatcatg cctacagccg ttgccgaacg taggctggat aaggcgttta cgccgcatcc 960
   ggcaattctc tgctcctgat gagggcgcta aatgccgcgt ggcctggaat tattgattgc 1020
   tcaaaccatt ttgcaaggct tcgatgctca gtatggtcga ttcctcgaag tgacctccgg 1080
   tgcgcagcag cgtttcgaac aggccgactg gcatgctgtc cagcaggcga tgaaaaaccg 1140
   tatccatctt tacgatcatc acgttggtct ggtcgtggag caactgcgct gcattactaa 1200
   cggccaaagt acggacgcgg catttttact acgtgttaaa gagcattaca cccggctgtt 1260
   gccggattac ccgcgcttcg agattgcgga gagctttttt aactccgtgt actgtcggtt 1320
   atttgaccac cgctcgctta ctcccgagcg gctttttatc tttagctctc agccagagcg 1380
   ccgctttcgt accattcccc gcccgctggc gaaagacttt caccccgatc acggctggga 1440
   atctctactg atgcgcgtta tcagcgacct accgctgcgc ctgcgctggc agaataaaag 1500
   ccgtgacatc cattacatta ttcgccatct gacggaaacg ctggggacag acaacctcgc 1560
   ggaaagtcat ttacaggtgg cgaacgaact gaagggcgaa ttctgcagat atccatcaca 1620
   ctggcggccg ctcgagcatg cat 1643